Europäisches Patentamt

(19)  European Patent Office

Office européen des brevets

(11)  EP 0 271 340 B1

## (12)  EUROPEAN PATENT SPECIFICATION

(45)  Date of publication and mention
of the grant of the patent:
**07.02.1996  Bulletin 1996/06**

(51)  Int. Cl.$^6$: **A61B 5/00**, A61B 5/14

(21)  Application number: **87310869.0**

(22)  Date of filing: **10.12.1987**

(54)  **Oximeter apparatus and method for measuring arterial blood constituents**

Messgerät und Verfahren zum Messen der arteriellen Blutsauerstoffsättigung

Oxymètre et méthode pour mesurer les constituants du sang artériel

(84)  Designated Contracting States:
**AT BE CH DE ES FR GB GR IT LI LU NL SE**

(30)  Priority:  **12.12.1986 US 940860**
**24.12.1986 US 946492**

(43)  Date of publication of application:
**15.06.1988  Bulletin 1988/24**

(73)  Proprietor: **Yelderman, Mark**
**Menlo Park  California (US)**

(72)  Inventor: **Yelderman, Mark**
**Menlo Park  California (US)**

(74)  Representative: **Baillie, Iain Cameron et al**
**D-80331 München (DE)**

(56)  References cited:
**EP-A-  102 816**          **EP-A- 0 160 768**
**WO-A-86/00514**        **DE-A- 2 136 823**
**US-A- 3 463 142**       **US-A- 4 167 331**
**US-A- 4 555 179**

• **MEDICAL AND BIOLOGICAL ENGINEERING, vol.
10, no. 1, January 1972, pages 9-22, Pergamon
Press, GB; F.J. JANSSEN: "The principle design
and features of a new Hb-oximeter"**

## Description

Field of Invention

This invention relates to the field of devices and methods for non-invasively measuring blood constituents which are capable of determining the hemoglobin oxygen saturation of blood and, more particularly, to devices which detect variations in the detected light amplitude of one or more wavelengths which are transmitted through body tissue and which compensate in some manner for ambient light interference.

Background of the Invention

The determination of blood oxygen and other blood constituents such as injected dyes has become a greater interest to physicians and of greater importance in the practice of clinical medicine. Generally, it is known to use spectrophotometric techniques to measure arterial hemoglobin oxygen saturation. Moreover, various blood constituent measuring devices and methods use non-invasive techniques wherein emitted light is passed through the sample, or reflected therefrom, to light sensors. Variations in the detected light at various wavelengths are then used to determine arterial oxygen saturation, and/or pulse rates. Such devices and/or methods are shown, for example, in U.S. Patent Nos. 4,407,290; 4,266,554; 4,167,331; 4,086,915; 3,998,550; and 3,647,299; and European Patent Nos. EP 0 104 771 A3 and EP 0 102 816 A3.

With respect to prior art oximetry devices and methods in particular, significant errors are induced in clinical oximetry if the classical absorption equation (Bier's Law) is used to calculate the saturation of oxygen as applicable to pure hemoglobin. Such methods are disclosed in U.S. Patent Nos. 4,167,331; 3,998,550; 4,086,915; and 4,266,554. Other patents teach the use of a mathematical approximation to Bier's Law by using ratios of the pulsating absorbance and the non-pulsating absorbance components of each of several wavelengths of transmitted light. (See, for example, U.S. Patent Nos. 4,407,290 and 3,647,299; and European Patent Nos. EP 0 104 771 A3 and EP 0 102 816 A3.) Because the light absorption of tissue does not exactly correspond to that predicted by Bier's Law, some type of empirical calibration may be performed as taught in U.S. Patent Nos. 4,407,290; 4,167,331; and 4,086,915.

The technique of deriving the absorbance in the pulsating component is taught in different ways. One technique relies upon the quantitative measurement in the change in absorbance at each wavelength. See, for example, U.S. Patent No. 4,407,290 and European Patent Nos. EP 0 104 771 A3 and EP 0 102 816 A3. It is also known that the derivative of the change in absorbance and a peak to peak measurement of the pulsating absorbance component may be used to calculate the oxygen content of arterial blood. See, for example, in U.S. Patent Nos. 4,407,290 and 4,167,331.

When a single light detector is used, the detected light for each wavelength must be separated. This is accomplished by using time separation and synchronous detection. See, for example, U.S. Patent Nos. 4,407,290; 4,266,554; and 3,647,299. Because the light detectors also detect ambient light, some type of ambient light rejection technique is normally employed. Some patents use four clock states and allow for substraction of ambient light. See, for example, U.S. Patent Nos. 4,407,290 and 4,266,554. Some patents use techniques to remove the non-pulsating absorbance component since ambient light is usually a non-pulsating absorbance frequency. See, for example, U.S. Patent Nos. 4,167,331 and 3,998,550. These techniques consider light to have a constant amplitude.

An article by F. J. Janssen entitled "The principle design and features of a new Hb-oximeter" and appearing in the journal Medical and Biological Engineering is concerned with a synchronous detection oximeter. European Patent Specification 160768 is concerned with spectrophotometric analysis primarily for glucose. Neither of these references compensates for white light noise and BOVIE interference by substantially matching driver and receiver circuitry within a narrow frequency range.

Although these techniques and devices have been utilized, they are not completely satisfactory and are deficient in several areas. Beer's Law and/or the use of empirical estimates usually only approximate the oxygen content of blood in living tissues in clinical environments. Prior techniques for removing ambient light and motion artifacts are unsatisfactory and generally produce decreased signal to noise rations and increased errors in the clinical measurements because up to fifty percent of the duty cycle is devoted to making ambient light measurements. Techniques using synchronous detectors are not completely satisfactory since they require wide bandwidth AC amplifiers and because they also may devote a significant portion of the duty cycle, as much as 50%, to measuring the ambient light rather than the desired absorbance changes. The wide bandwidth requirements of the prior art devices are more susceptible to frequency interference such as BOVIE interference. BOVIE interference is a type of system noise produced by electrical surgical devices such a coagulators and cauterizers. It typically effects the frequency range of .5 to 5 MHZ, but can be found even in direct current. While there is less energy in frequencies between DC and .5 MHZ, there is still enough energy to potentially cause interference which impairs the performance of many prior art devices. Previous embodiments also frequently require an analog channel for each wavelength and require that these channels be matched over the bandwidth. The analog requirements may be so stringent as to require that both channels have a "normalized" DC output. See, for example, U.S. Patent No. 4,407,290.

While blood constituents measuring devices and methods have heretofore been suggested and have achieved some success, a need still exists for more accurately measuring the oxygen saturation of the blood in anemic patients particularly those with blood conditions, such as low red blood cell counts. These blood counts are different from the assumed counts upon which the factory "pre-sets" are based. Correcting for anemia in the saturation equations, for example, could be a critical factor in keeping an anemic's brain and heart alive during surgery. These is also a need for a device which is less sensitive to other types of interferences, such as BOVIE interference, which can virtually interrupt oximetry at critical times during operation.

According to the present invention there is provided a blood constituent measuring device for non-invasive measurement of the concentration of a blood constituent, as defined in claim 1.

According to a further aspect of the invention there is provided a method for non-invasively measuring the concentration of a blood constituent for use in an environment generally illuminated by artificial light including the steps defined in claim 6.

A novel blood constituent measuring device and method are provided which exhibit an excellent signal to noise ratio and improved resistance to interference from ambient artificial light, and BOVIE interference, etc. This is accomplished by driving one or more light sources with a driving signal. This driving signal, does not exhibit a frequency which is present in the artificial light environment of the operating room. The light transmitted through the body tissue is then received by a receiver means which is tuned to the preselected driving signal to produce a demodulated output. As a result, no portion of the duty cycle must be devoted to measuring ambient light, and the narrow band pass filters employed reduce BOVIE interference.

The present invention also provides novel techniques for determining and displaying pulse and oxygen saturation values. Blood oxygen levels are calculated using equations which include red cell scattering parameters, and measurements may be corrected for abnormal red cell counts. The device also utilizes other novel signal processing techniques, including digital analysis and interpolation of temporally distinct detected light amplitudes.

It is, therefore, an object of this invention to provide a blood constituent measuring device and method.

It is another object of this invention to provide a blood constituent measuring device and method capable of determining the oxygen saturation of arterial blood in relation to the fraction of red blood cells per unit volume of whole blood.

It is still another object of this invention to provide a blood constituent measuring device and method that can provide an output which indicates arterial pulse.

It is still another object of this invention to provide a blood constituent measuring device and method that rejects motion artifacts by subtracting extraneously detected data from a detected pulse data reading.

It is still another object of this invention to provide a blood constituent measuring device and method that calculates the oxygen saturation of arterial blood using red blood cell absorbance and scattering parameters.

With these and other objects in view, which will become apparent to one skilled in the art as the description proceeds, this invention resides in the novel construction, combination, arrangement of parts, and methods substantially as hereinafter described and more particularly defined by the attached claims.

Brief Description of the Drawings

The accompanying drawings illustrate a complete embodiment of the invention according to the best mode so far devised for the practical application of the principles thereof, and in which:

FIG. 1 is a process flow diagram for an oximeter utilizing in this invention;
FIG. 2 is a software flow diagram showing digital to analog conversion points for interfacing with the oximeter's hardware, as well as processing steps for sampling light intensity readings, storing said readings, and calculating saturation values for hemoglobin in patients' blood;
FIG. 3 is a detector block flow diagram indicating the elements of the detector and receiver circuitry and possible locations for adjusting the amplifier gain;
FIG. 4 is a light-emitting diode driver flow diagram indicating how a preferred square wave driving signal is generated, with optional adjustment to drive current and an optional calibration verification procedure;
FIG. 5 is a schematic of the light emitting diode driver circuitry;
FIG. 6 is a schematic of the detector circuitry;
FIG. 7 is an exemplary bar graph, showing a room light spectrum of the estimated amplitudes of interfering ambient artificial light in a hypothetical environment where the power line frequency is a nominal 60 cps; the actual relative amplitudes of such interference and their frequencies varying somewhat depending upon power line frequency, lighting source, etc.

Description of the Invention

The following description is provided to enable any person skilled in the medical and electronic fields to make and use the invention and sets forth the best mode contemplated by the inventor of carrying out his invention. Various modifications, however, will remain readily apparent to those skilled in the art since the generic principles of the present invention have been defined herein specifically to provide a relatively economical and easily manufactured non-invasive blood constituent measuring device.

The invention herein described includes at least one light source driven with a preselected driving signal, which source is placed in proximity to and directed through a patient's body tissue, a photodetector for receiving the light transmitted through that tissue, and a receiver circuit tuned to the driving frequency of the light source. The device effectively excludes ambient light, and limits BOVIE interference and system noise by driving the light source with a signal having a frequency not found in the ambient light environment. A processing means processes the receiver circuit output and calculates blood saturation using scattering parameters which are proportional to the red cell count in the tissue.

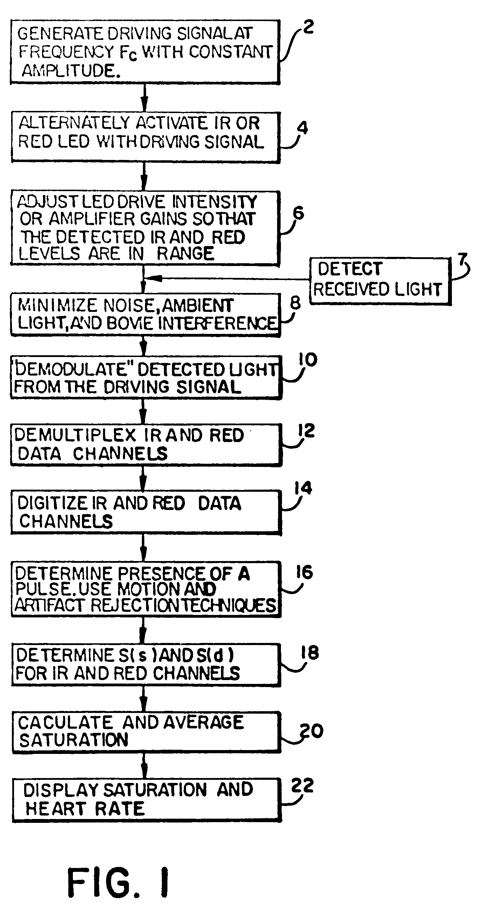

Referring now to the drawings, Figure 1 is a process diagram of an oximeter utilizing the instant invention. A driving signal having a pre-selected waveform is generated at process step 2. The driving signal is used to alternately activate the light emitting diodes at step 4. An adjustment of the light emitting diode drive intensity and/or amplifier gains can be made at step 6, so that the detected light levels 7 are within the range of the analog to digital converters used in the hardware. Noise, ambient light, and BOVIE interference are then minimized at step 8. The detected light signal is then demodulated from the driving signal at step 10. Demultiplexing the data channels is then accomplished at step 12, and the output of step 12 is digitized at process step 14. The data is then processed by determining the presence of a pulse and implementing motion and artifact rejection techniques at step 16. Saturation values are then calculated for each wavelength of light a step 18, and an average value for saturation is determined at step 20. Finally, the saturation and heart rate values are displayed at step 22.

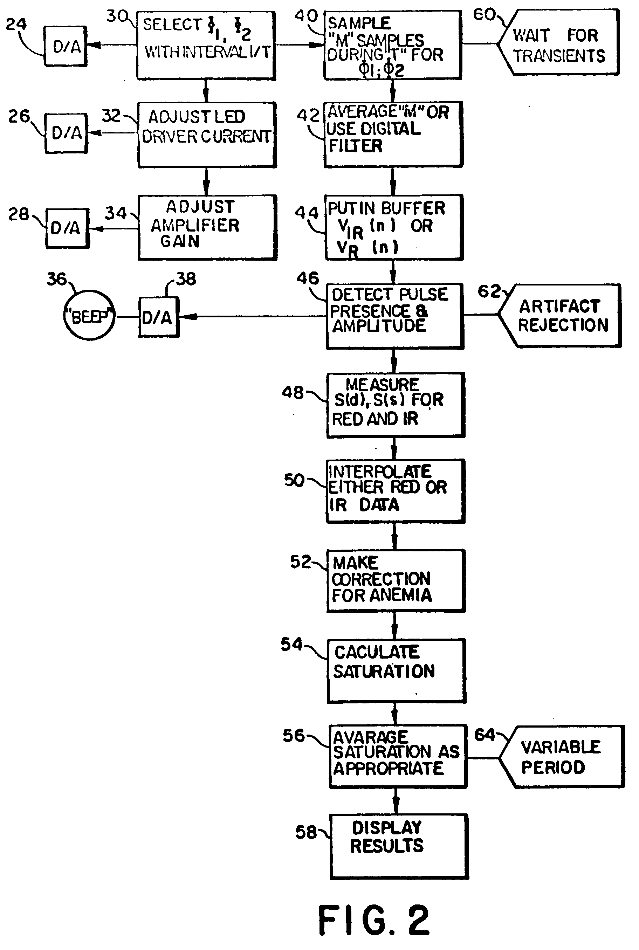

Referring now to the software flow diagram in Figure 2, the driving signal in step 2 above which preferably has a uniform amplitude and frequency, such as a square or a sine wave, is selected to drive a pair of light emitting diodes at a predetermined clock state, designated "$\Phi_1$" or "$\Phi_2$", at software step 30. Adjustments to the diodes' driver current and the oximeter's amplifier gain can be made at steps 32 and 34, respectively. Before data points representing transmitted light values are sampled at step 40, the software enables system transients to settle at step 60. The delay necessary to overcome transients in the waveform during a given clock state will vary according to the wave generating circuit used and the amount of duty cycle required for measuring data points. In the preferred embodiment no more than 25% of the waveform generated by the driver circuit should be used for transient settling. Preferably, less than 10%, and most preferably, less than 5% of the waveforms should be allocated for waiting for transients to settle. Alternatively, the settling time can be terminated when the transient amplitude comprises less than 1%, and preferably less than .1%, of the amplitude of the theoretical waveform. The sample points designated as "M" are then either averaged or filtered by using a digital filter at software step 42. The averaged or filtered data points are then placed into a buffer at software step 44, for enhanced signal to noise ratios and better analog to digital resolution.

Detecting the pulse presence and amplitude in software step 46 is accomplished by first measuring the light intensity readings for the preferred red and infrared wavelengths at software step 48. Values at step 48 may be at peaks or at other times in the cycle, but should be measured or estimated at a synchronous point in time. Since alternate clock states are preferred, an interpolation at step 50 is used for this purpose. By interpolating measured values of the light detected from one light-emitting diode to estimate a detection value when said diode is not illuminated, the device can render the clock states, $\Phi_1$ and $\Phi_2$ synchronous.

An anemia correction calculation is then made at software step 52. The correction for anemia is made by incorporating into the saturation calculations, a factor "$\eta$" which is equivalent to the fraction of red cells per unit volume of whole blood which is calculated from the hemoglobin concentration.

The software process next encompasses calculating the saturation value "S" at step 54. An average saturation value may be calculated as appropriate at step 56, and the results are displayed at step 58.

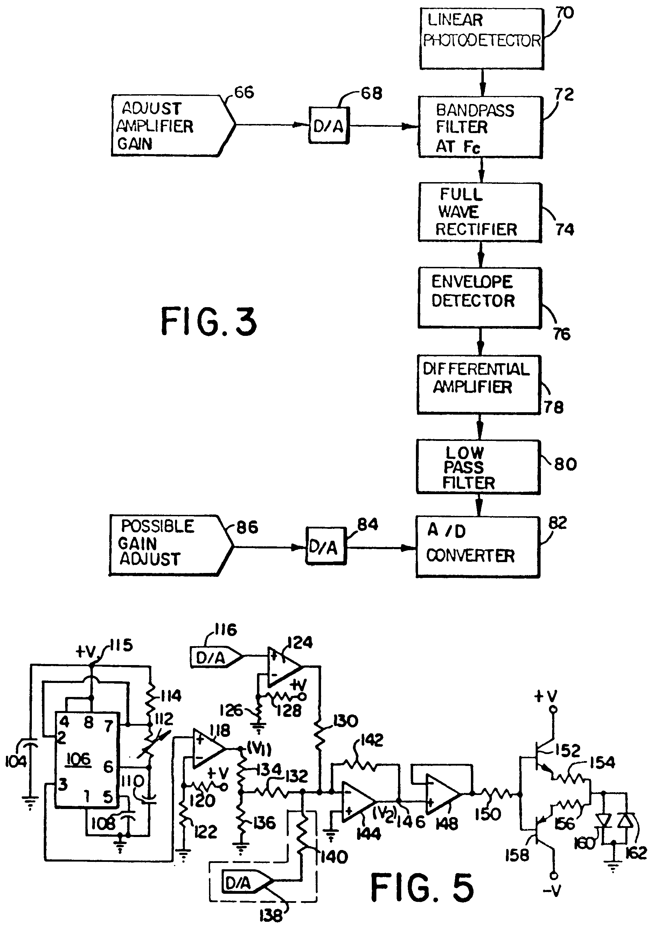

Referring next to the detector flow diagram in Figure 3, software step 34 operates to adjust the amplifier gain through the digital to analog converter 68. The gain adjustment is made in the band pass filter element at block 72. This band pass filter receives the output from the linear photo detector of block 70, and operates with the whole wave rectifier, block 74, and envelope detector, block 76, to form an AM demodulator. These hardware elements carry out the process steps of eliminating interference and demodulating the detected light values from the driver frequency, process steps 8 and 10 of Figure 1. A differential amplifier follows next in block 78 which adds both parts of the whole wave rectifier output together. The output of the differential amplifier is then fed into a low pass filter at block 80 which is intended to eliminate ripple from the carrier frequency and generally improve the signal to noise ratio. The preferred embodiment also permits a possible gain adjustment at block 86 to keep the analog signal appropriate for the analog to digital converter, block 82.

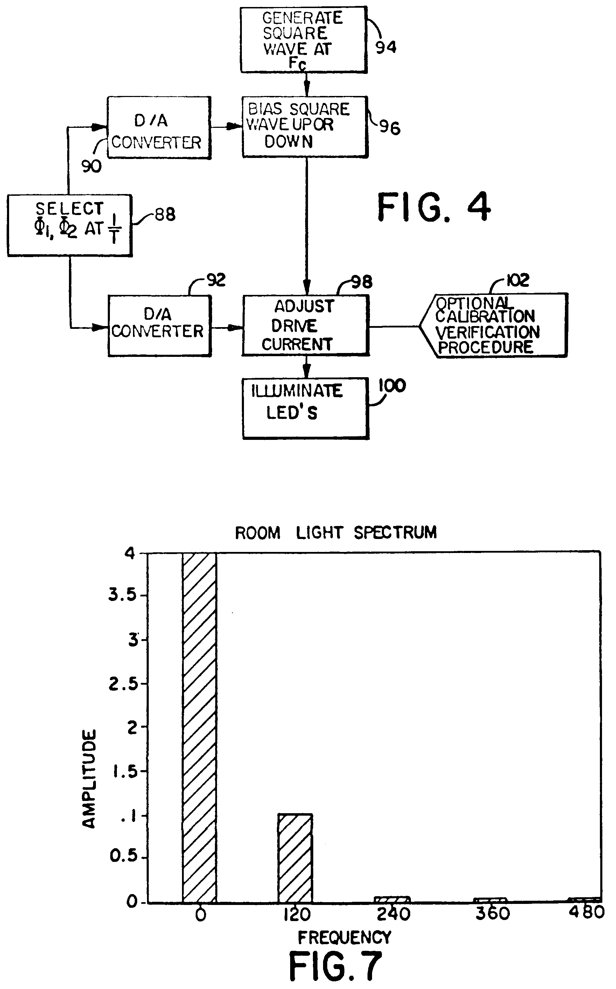

Referring next to the driver flow diagram found in Figure 4, a driving signal having a pre-selected waveform, as previously discussed, is generated at block 94 which corresponds to process step 2. The resulting driver waveform can be biased up or down by block 96 to drive back to back LED's. This biasing step may also be replaced by a switching means for alternatively driving each light-emitting diode. It is also noted that software step 30 operates through digital to analog converters 90 and 92 to determine the duration of excitation of the light emitting diodes. Software step 32 also operates on the driver circuit to determine the intensities of the illuminated light emitting diodes in block 100. Block 102 represents an optional calibration verification procedure which can be adapted to this preferred embodiment.

When only one driving signal is used to drive two light-emitting diodes, the light-emitting diodes are driven alternatingly by that signal. The duration of excitation of each light-emitting diode is known as the clock-state and is designated as "T". If two light-emitting diodes are used, two clock-states are selected and are designated as $\Phi_1$ and $\Phi_2$. It is contemplated that the light-emitting diodes can be driven using signals such as decaying waveforms or other coded waveforms. These are not presently preferred since tuning the recurring means to, or decoding, those waveforms is somewhat more complicated. In any event, the driving signal does not substantially overlap with the substantial artificial light amplitudes in the intended location of use.

The light-emitting diodes should be kept as bright as possible to improve the signal to noise ratio of the device. However, at least one light-emitting diode may require adjusting. The light-emitting intensities are intentionally not adjusted to be equal.

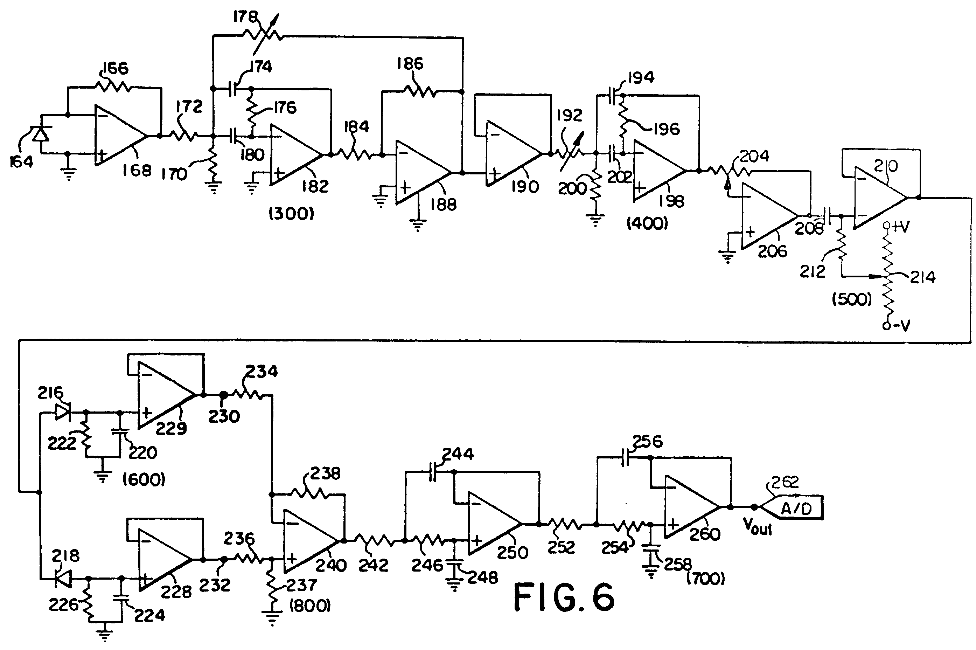

Referring next to the light-emitting diode driver schematic found in FIG. 5, a "555" integrated circuit 106 is used to generate a driver frequency. The present configuration produces a uniform square wave at 47kHz. It is desirous that the carrier frequency be as high as possible to optimize settling times of the oximeter's circuitry.

However, it may also be desirable to choose frequencies which are located below the frequency of the ambient light or in between the harmonics of the ambient light. As a minimum requirement, "a frequency not substantially present in the ambient light" means that the frequency is not within an amplitude which comprises greater than 1% of the amplitude of the ambient light. Moreover, "in between frequencies" may be defined as within a range delineated by two principle harmonic frequencies, plus or minus 10% of said harmonic frequencies.

Using the 60 cycle per second frequency found in the power lines of the United States, one could choose a carrier frequency of less than 60 cycles per second, in between the harmonics of this frequency: 120, 240, 480, and 960 cycles per second, or greater than an arbitrary cutoff point of power line harmonic interference. (See Figure 7 depicting a hypothetical ambient light spectrum with frequencies of 120, 240, 360 and 480 cps.) In the present embodiment, a carrier frequency above the fourth harmonic would ideally avoid most ambient light harmonic interference.

This principle is equally applicable to power line frequencies outside the United states, such as 50 to 55 cycles per second found in Europe. Several examples of selected frequency ranges are herein provided to serve as a reference:

Example 1: Possible ranges for carrier frequencies for a power line frequency of 60 cycles per second.

1. Less than 54 cycles per second.
2. Between 66 and 108 cycles per second.
3. Between 132 and 216 cycles/second.
4. Between 264 and 432 cycles/second.
5. Above 1056 cycles/second.

Example 2: Possible ranges for carrier frequencies for a power line frequency of 50 cycles per second.

1. Less than 45 cycles/second.
2. Between 55 and 90 cycles/second.
3. Between 110 and 180 cycles/second.
4. Between 220 and 360 cycles/second.
5. Above 880 cycles per second.

Example 3: Possible ranges for carrier frequencies for a power line frequency of 55 cycles per second.

1. Less than 49.5 cycles/second.
2. Between 60.5 and 121 cycles/second.
3. Between 121 and 198 cycles/second.
4. Between 242 and 396 cycles/second.
5. Above 968 cycles/second.

Furthermore, in this driver schematic, the voltage at resistor 134 which we will call "$V_1$" is in the form of a square wave at a fixed carrier frequency with the maximum voltage equal to the voltage across resistor 128 and a minimum voltage equal to 0 volts.

Output from the digital to analog converter 116 is either +10 volts of 0 volts and is used to determine clock states $\Phi_1$, $\Phi_2$ selected at software step 30 in Figure 2. For this embodiment, a Computer Continuum LAB 40 with a LAB 40-2 12-bit analog to digital module is used. Bit 0 of the 8-bit data output is connected to the operational amplifier 124. A summation operation amplifier configuration 144 is used to combine the carrier frequency voltage across resistor 132 with the offset voltage across resistor 130 generated by the digital to analog converter 116. The output voltage at 146 is a square wave with a frequency $F_c$. At $\Phi_1$, the maximum voltage equals 10 volts, the minimum voltage equals 0 volts or at $\Phi_2$, the maximum voltage equal 0, and the minimum voltage equals -10 volts. A buffer operation amplifier 148 follows.

The light-emitting diode current driver section follows the buffer 148. Power transistors 152 and 158 are used. For this embodiment, transistor 152 is a TIP 31 and transistor 158 is a TIP 32. Load resistors 154 and 156 are used to determine light-emitting diodes 160 and 162 current and brightness.

Generally, the physiology requires that the red light-emitting diode be about twice as bright as the infrared light-emitting diode. Thus, resistor 154 is chosen to deliver about 100mA to the red light-emitting diode and resistor 156 is chosen to deliver about 50mA to the infrared light-emitting diode. Attenuating the infrared light-emitting diode to about 50% yields both intensities near each other at the detector. This is for convenience, and not a requirement. The two light emitting diodes 160 and 162 are connected in opposite directions. Thus, a current driven in one direction will illuminate one of the light-emitting diodes and a current in the opposite direction will illuminate the other, producing a pulsing effect. Since the opposed relationship is not a requirement, this device could also employ separate wirings for each LED.

A later embodiment of this light-emitting driver circuit encompasses a calibration verification device shown by the digital to analog converter 138 and resistor 140. A small sine signal at 1Hz or other suitable waveform, such as one emulating an arterial pulse, would produce a signal which would be interpreted at the detector as a pulse. Resistor 140 could be any value to keep the scale appropriate.

This embodiment also utilized a voltage across resistor 128 of +12 volts. Also included in this embodiment was an adjustment made at rheostat 112 so that the fixed carrier frequency was equal to 47kHz. The operation amplifiers 118, 124, 144 and 148 in this configuration are BiFets, TLØ74 and the light-emitting diodes used in this embodiment are from a Nellcor D-25 Oxisensor. A linear photo-detector 70 of the detector flow diagram Figure 3, is used for detecting the transmitted light from the body tissue of a patient. A linear photo detector is used so that the output of the photo-detector is directly proportional to the input as represented by the light emission intensities. For this embodiment, the photo detector used is from a Nellcor D-25 Oxisensor. Capacitor 104 is 88 μf. Capacitors 108 is .1 μf and 110 is .01 μf. Resistors 120, 122, 126, 134 and 136 are 10KΩ. Resistors 132, 142 are 100 K Ω and 150 is 100 Ω. Resistor 114 is 135Ω. Resistor 112 is variable. Resistor 128 is 46 KΩ. Resistor 130 is 220 K Ω. Resistor 140 can be preselected. Resistor 154 is 100 and resistor 156 is 200.

Referring next to the detector electrical schematic in Figure 6, the photo detector 70 is connected directly to an operation amplifier as represented by diode 164, resistor 166 and operation amplifier of FIG. 6.

The next stage is a band pass filter circuit, 300 and 400, of FIG. 6 designed with a center frequency at the carrier frequency. For this embodiment, two band pass filters were used, 300 and 400. The first band pass filter 300 had a gain equal to 10 and Q equal to 50. The second band pass filter 400 has a gain equal to 4, but variable, and a Q equal to 10. This plurality of band pass filter states enables the oximeter to filter out noise frequencies while at the same time accepting only the frequency associated with the admitted light intensities. This frequency filtering state acts much like a tuner on a radio in that the band pass filters are matched or tuned to the carrier frequency. The use of a narrow bandwidth devices represents a significant improvement over the wide band AC amplifiers of the prior art.

By using a narrow band width amplifier as opposed to a wide band, BOVIE interference, created by electronic surgical instruments which can sufficiently cause interference in a range less than 1kHz, can be minimized. BOVIE interference, which typically effects the frequency range of .5 to 5 mHz; can be found even in direct current. Wide band amplifiers pick up the BOVIE signal more readily, thereby, creating more error in the clinical environment. A narrow band amplifier like the one used herein has a much greater chance at eliminating BOVIE interference and can produce a better signal to noise ratio.

A variable gain direct current inverting amplifier represented by resistor 204 and operation amplifier 206 follows the band pass filter 400 to allow the user to adjust the overall system gain as appropriate to yield an output voltage which is approximately 8 volts for the larger voltage channel.

A calibration adjustment 500 is found in the detector circuitry next. The 3000 pF capacitors 220 and 224 can be removed and voltages on the rectifier 214 are adjusted until equal using this trimpot procedure.

A full wave rectifier 600 follows. Time constants on the envelope detectors located within the full wave rectifier 600 should be less than 1 msec. or less than 10% of "T". This would allow a complete settling time of about 4 msec. or 40% of "T". This allows approximately 60% of "T" for sampling data points. Preferably the time constant is less than 0.25 in sec. or less than 2.5% of "T". This would allow a complete settling time of 1 msec. or 90% of "T". Resistors of 270 KΩ, 222 and 226, and capacitors of 3000 pF, 220 and 224, yield 1 msec. time constant. Additional settling time requirements may be imposed by the low pass filter section 700.

The time constants for the envelope detectors are inversely proportional to amount of ripple from the carrier frequency such that the smaller the time constant, the larger the ripple detected. Although the ripple can be averaged out later, it will affect accuracy of the instrument to some extent.

The ripple (r) is calculated as a function of the driving frequency, the resistance in the envelope detector and the capacitance in the envelope detector. See R.E. Smith, Circuits, Devices, and Systems, Wiley, New York, Second Edition, 1967, p. 429.

The bandpass filter ciruit, full wave rectifier, and envelope detectors in combination, operate on the driver wave frequency to recover the waves representing detected light intensity values which were previously modulated with the driving frequency.

A differential amplifier 800 follows which consists of resistors 234, 236, 237, 238, and operational amplifier 240. For this embodiment, the differential amplifier should have matched components; here they are shown as tolerances of 1%.

The low pass filter sections 700 follows. Two identical low pass filters are shown in series. The low pass filter should have a cutoff frequency low enough to reject ripple from the rectifier by at least -100 dB, yet not so low that the higher harmonics of the pulse wave form are rejected. The other requirement is that the rise time or setting time be fast enough, in the same order as the rectifier circuits 600, so that adequate time is available at the end of each clock state for sampling data points.

The minimum settling time filters used in this preferred embodiment are BESSEL filters with damping factors, $\zeta$, equal to 0.8659. A two-pole BESSEL filter is preferred with a cutoff frequency of 250Hz with 98% settling by 1 ms.

The output voltage from the second low pass filter represented by resistor 252, capacitor 256, resistor 254, capacitor 258 and operational amplifier 260 is connected directly to the analog to digital converter 262. For this embodiment, a COMPUTER CONTINUUM LAB 40 with a LAB 40-2 12-bit analog to digital module is used.

The output data from the preferred embodiment circuitry is used to calculate a pulse rate and saturation values. The following variables are used in the software program found herein and summarized in Figure 2:

$\Phi_1$, $\Phi_2$: clock states or periods of excitation of light-emitting diodes;

M: the number of analog to digital conversions or sample data points during a $\Phi$ state lasting for T;

T: the duration of a clock state, $\Phi$;

$V_{ir}$: the voltage intensity reading as measured by the photo detector from the infrared light-emitting diode;

$V_r$: the light intensity reading as measured by the photo detector from the red light-emitting diode;

S[d]: the light intensity measured at diastole and is the virtual incident intensity for pulse oximetry;

S[s]: the light intensity measured at systole;

S: the fractional oxygen saturation of hemoglobin.

The operating principles of the present invention will now be described. Hemoglobin is actually a concentration of oxyhemoglobin and a concentration of deoxyhemoglobin. Because there are two species present, two different and unique wave-lengths of light must be used to generate two simultaneous equations. A minimum of one wavelength is required for the measurement of each separate blood constituent component. These equations which model the extinction of light by clinical oximeters as a function of absorbence and scattering. Since scattering is a function of wavelength, hemoglobin concentration and saturation, including scattering terms decreases the amount of errors which have caused previous oximeters, which relied on absorbence factors, to be inaccurate in certain clinical environments. (See example, Joseph M. Schmitt, Optical Measurement of Blood Oxygen by Implantable Telemetry, 31, 41 (February, 1986), Standard Electronics Laboratories, (Describing scattering coefficients in the implantable telemetry art.)

The teaching of the thesis entitled "Optical Measurement of Blood Oxygen by Implantable Telemetry" by Joseph M. Schmitt is relevant to the discussion of calculating oxygen saturation of hemoglobin and is hereby incorporated by reference into this specification.

The general function for the total observed light at an observation point is given by the following equation:

$$S(p) = \Psi_0 \, \Sigma_{st} \exp(-\Sigma_t z) R(r), \tag{1}$$

where

S(p) = source function or amount of detected light;

$\Psi_0$ = incident light flux;

R(r) = function describing radial distribution of light beam intensity which is assumed to equal 1 for this discussion.

The more useful saturation determination is arterial hemoglobin saturation. Because at systole there is a rapid inflow of arterial blood, measuring the change in light extinction from late diastole to early systole will facilitate this measurement. These types of oximeters are designated pulse oximeters. For pulse oximetry, S[p] assumes values between S[d], intensity at late diastole, and S[s], intensity at early systole. S[s] corresponds to the intensity measured during systole and is the transmitted intensity for pulse oximetry. S[d] corresponds to the intensity measured at late diastole and is the virtual incident intensity for pulse oximetry. The optical path, or incremental change because of pulsation or the inflow of arterial blood, also changes yielding a value of z' at diastole and z" at systole. Then two equations can be written for diastole and systole.

$$s(d) = \Psi_0 \, \Sigma_{st} \exp(-\Sigma_t 2') \qquad (2)$$

$$S(s) = \Psi_0 \, \Sigma_{st} \exp(-\Sigma_t z'') \qquad (3)$$

The difference in extinction between diastole and systole is due primarily to inflow of arterial blood.

$$S(d)-S(s) = \Psi_0 \Sigma_{st}[\exp(-\Sigma_t z')-\,_{exp}(-\Sigma_t z'')]. \qquad (4)$$

But, z'' equals z' + z where z is the incremental change due to pulsatile flow. Then the equation can be rewritten as

$$S(d)-S(s) = \Psi_0 \Sigma_{st}[\exp(-\Sigma_v z')-\exp(-\Sigma_t z')\exp(-\Sigma_t z)]; \qquad (5)$$

$$S(d)-S(s) = \Psi_0 \Sigma_{st}\exp(\Sigma_t z')[1-\exp(1\Sigma_t z)]. \qquad (6)$$

For any in vivo transducer application, the terms $\Psi_0$, $\Sigma_{st}$, and $\exp[-\Sigma_t z']$ will be a constant for a particular clinical situation which we will designate as K for now. Then the equation becomes:

$$S(d)-S(s)=K[1-\exp(-\Sigma_t z)]. \qquad (7)$$

Now it becomes necessary to develop the terms of $\Sigma_t$.

$$S(d)-S(s)/K=[1-\exp(-(\sigma_{ao}S+\sigma_{ar}(1-S)+\Sigma_{st}/\eta)\eta z)] \qquad (8)$$

$$[1-(S(d)-S(s)/K)]=\exp(-(\sigma_{ao}S+\sigma_{ar}(1-S)+\Sigma_{st}/\eta)\eta z) \qquad (9)$$

In the above equation, only two variables, S and z, are present and the other terms are constants, some being wavelength dependent. The next step is to take logarithms.

$$\ln[1-(S(d)-S(s))/K]=-(\sigma_{ao}S+\sigma_{ar}(1-S)+\Sigma_{st}/\eta)\eta z \qquad (10)$$

$$-\ln[1-(S(d)-S(s))/K]=(\sigma_{ao}S+\sigma_{ar}(1-S)+\Sigma_{st}/\eta)\eta z \qquad (11)$$

$$-\ln[1-(S(d)-S(s))/K]/\eta z=(\sigma_{ao}S+\sigma_{ar}(1-S)+\Sigma_{st}/\eta) \qquad (12)$$

$$(\sigma_{ar}-\sigma_{ao})S-\ln[1-(S(d)-S(s))/K]/\eta z=\Sigma_{st}/\eta+\sigma_{ar} \qquad (13)$$

Because there are two variables, two equations or wavelengths are needed.

$$\text{wavelength 1: } -\ln[1(S_i(d)-S_i(s))/K_i]/\eta z+ (\sigma_{ari}-\sigma_{aoi})S=\Sigma_{sti}/\eta+\sigma_{ari} \qquad (14)$$

$$\text{wavelength 2: } -\ln[1-(S_{ii}(d)-S_{ii}(s))/K_{ii}]/\eta z+ (\sigma_{arii}-\sigma_{aoii})S=\Sigma_{stii}/\eta+\sigma_{arii} \qquad (15)$$

where
$\sigma_{ao}$ is equal to absorption cross section of an isolated red cell containing completely oxygenated hemoglobin;
$\sigma_{ar}$ is equal to absorption cross section of an isolated red cell containing completely deoxygenated hemoglobin;
$\sigma_s$ is equal to scattering cross section;
$\mu$ is equal to an asymmetry parameter;
$\eta$ is equal to fraction of red cells per unit volume of whole blood;
H is equal to hematocrit fraction of whole blood;
$\Sigma_{st}$ is equal to modified scattering coefficient of whole blood;
$\Sigma_s$ is equal to scattering coefficient of whole blood;
$\Sigma_a$ is equal to absorption coefficient of whole blood;
$\Sigma_t$ is equal to sum of modified scattering and absorption coefficients of whole blood;
z is equal to distance or curvette length;
$\Sigma_a$ is equal to $\eta[\sigma_{ao}S + \sigma_{ar}(1-S)]$;
$\Sigma_s$ is equal to $\eta \, \sigma_s(1-H)$;
$\Sigma_{st}$ is equal to $\Sigma_s(1-\mu)$;
$\Sigma_t$ is equal to $\Sigma_a + \Sigma_{st}$;

$\Sigma_t$ is equal to $\eta[\sigma_{ao}S + \sigma_{ar}(1\text{-}S)] + \Sigma_{st}$; and

K is equal to constant for the product of the source function, incident light flux, and exp $(-\Sigma_{tz})$.

These are two simultaneous equations in variable S and 1/z. The four measurements: $S_i[d]$, $S_i[s]$, $S_{ii}[d]$ and $S_{ii}[s]$ are made. The hemoglobin concentration is used to calculate $\eta$. Correcting the concentration formulae for the number of red blood cells per unit volume of whole blood is a novel approach to problems associated with anemia which had been ignored by the prior art. The above equations utilize this important factor to calculate the oxygen content thereby providing a more accurate means to measure blood constituents of anemic patients. The following table is used which displays the other constants needed for two hypothetical wavelengths, 910nm and 660nm.

|  | 910nm | 660nm |
|---|---|---|
| $\sigma_{ao}$ | .1340 | .0357 |
| $\sigma_{ar}$ | .0802 | .3547 |
| $\sigma_s$ | 36.34 | 60.65 |
| $\sigma_{st}$ | .276 | .309 |
| $\mu$ | .9924 | .9949 |

The variable K includes gain factors, but in particular contains the overall gain factor which will be different for each wavelength channel and will require determination depending upon hardware. Also a unit conversion factor must be included since the variables in K contain physiology and measurement parameters, such as S(d). Thus S(d) and S(s), in whatever units they happen to be must be converted to physiology terms, i.e. the units in the log expression must cancel. Note further $K_i$ and $K_{ii}$ are neither equal nor do they render $S_i(d)$ and $S_{ii}(d)$ to be equal, or $S_i(S)$ and $S_{ii}(S)$ to be equal.

These equations can be solved by methods of linear algebra to determine S. Using techniques of matrix manipulation, a determinate is made and then the calculation of S.

The computer program receives the output signals from the analog to digital converter 262 and calculates the oxygen saturation [S] in the blood according to equations (14) and (15). The operation performing the solving of the selected equations amounts to multiplying, dividing, adding and subtracting utilizing the received variable and the stored constants.

The program used in this preferred embodiment is written in BETTER BASIC [Summit Software Technology, Inc., 1984] and is attached to this specification. The software has interfacing points with the analog hardware at digital to analog converters 1, 2 and 3 of FIG. 2. The program alternates between the two clock states for setting the output latch digital to analog converter 45, to 10 volts or ground, waiting to the end of the state and sampling only one data point [M = 1] 8.

Using an IBM-XT, the program in this preferred embodiment produces a clock state duration, T, of 12ms. 8. A buffer 10 of a thousand data points are taken for the red and the infrared channels. The buffer 44 is recorded onto a disc for examination by the higher level of software calculations 11 through 16.

The processing next encompasses calculating the saturation value S using the equations (14) and (15) as previously discussed. An average saturation 16 may be calculated as appropriate at this point in the processing.

As seen from above, the driving signal used to vary the amplitude of LED output within each clock state is not a clocking signal, i.e. it does not function to alternatively activate two or more LED's. In the present device a second signal produced by the clocking means is superimposed as the driving signal for performing this function. Increases in the frequencies of prior art clocking signals would not accomplish the results disclosed herein because such increases would require the use of extremely wide band amplifiers in the receiver, and thus would not improve, and may decrease, the signal to noise ratio of the device.

The following program writting in BETTER BASIC [Summit Software Technology, Inc., 1984], is used in the preferred embodiment of this invention.

```
SOURCE
PROCS=0
REAL:  PA0, PA1, PB, PC, CO, CW, LBYTE, HBYTE
REAL:  VALUE, J
REAL ARRAY (1000):  RED, IR
REAL:  I, X
    10  'SAMPLE.BAS:  23-AUG-1986: MARK YELDERMAN
    20  'BETTER BASIC FOR IBM-XT
    30  'COMPUTER CONTINNUM USING LAB 40-2 A/D
CONVERTER, 12 BITS
    40  '-----------------------------------------
    50  '*****************************************
    60  'Definition of LAB 40 Port addresses
    70  'PAO=736:PA1-737
    80  'PB=740: PC=744: CO=748
    90  'CW = 192  CONTROL WORD FOR PORT A BI-
DIRECTIONAL AND PORT B OUT
    100  'CLS
    110  'OUT (CO, CW) DEFINE LAB 40 PORT DIRECTIONS
    120  'OUT (PC, 2) SET UP BOARD SELECT 2 TO ENABLE
12 BIT MODULE
    130  'THE LAB 40 BUS IS NOW READY TO TALK TO THE
12 BIT MODULE
    140  'NOW TAKE DATA
    150   FOR J = 0 TO 999
    160  OUT (PA1,1)  'CHANGE OUTPUT STATE BY
STARTING A/D
    170  'DO MATH FOR PREVIOUS CONVERSION
    180  VALUE = HBYTE*256+LBYTE  COMPUTE 12 BIT
VALUE
    190
    200  RED(J)=VALUE  'RESULTS FOR STATE "O"
    210  FOR I=1 TO 2:X=I:NEXT  'THIS IS A DELAY LOOP
    220  OUT (PAO,1)  'START A/D CONVERSION WITH AN
OUPUT INSTRUCTION
    230  OUT (PB,O)  'SET BYTE SELECT TO READ LOW
BYTE
```

```
240  LBYTE=INP(PAO):LBYTE=INP(PAO)  'READ IN LOW
BYTE FROM PORT A
250  OUT (PB,1)  'SET BYTE SELECT TO READ HIGH
BYTE
260  HBYTE=INP(PAO):HBYTE=INP(PAO)  'READ IN HIGH
BYTE
270  '
280  OUT (PA1,O)  'CHANGE OUTPUT STATE BY
STARTING A/D
290  'DO MATH FROM PREVIOUS CONVERSION
300  VALUE = HBYTE*256+LBYTE  'COMPUTE 12 BIT
VALUE
310  '
320  IR(J)=VALUE  'RESULTS FOR STATE "1"
330  FOR I=1 TO 2:X=I:NEXT  'THIS IS A DELAY LOOP
340  OUT (PAO,O)  'START A/D CONVERSION WITH AN
OUTPUT INSTRUCTION
350  OUT (PB,O)  'SET BYTE SELECT TO READ LOW
BYTE
360  LBYTE-INP(PAO):LBYTE=INP(PAO)  'READ IN LOW
BYTE FROM PORT A
370  OUT (PB,1)  'SET BYTE SELECT TO READ HIGH
BYTE
380  HBYTE=INP(PAO):HBYTE=INP(PAO)  'READ IN HIGH
BYTE
390  NEXT
400  OPEN "TEST.DAT" FOR OUTPUT AS #2
410  FOR J=O TO 999
420  PRINT #2 USING "####.# ####.#"; RED(J),
IR(J)
430  NEXT
440  CLOSE
450  FOR J=O TO 99
460  PRINT USING "####.# ####.#"; RED(J),IR(J)
470  NEXT
ENDFILE
```

A list of components which have been utilized in a working embodiment of this invention is set forth hereinafter. It is to be realized, however, that the invention is not meant to be limited to the components as listed. The component list is as follows:

| Transistors: | 152-TIP 31; 158-TIP 32. |
| --- | --- |
| Light-Emitting Diodes: | 160 and 162 taken from a NELLCOR D-25 oxisensor. |
| Linear Photodetector: | taken from a NELLCOR D-25 oxisensor. |
| Operation Amplifiers: | TLØ74 BiFets |

Resistors: 112-variable; 114-135; 120-10K; 122-10K; 126-10K; 128-46K; 130-220K; 132-100K; 134-10K; 136-10K; 142-100K; 150-100; 154-100; 156-200; 166-470K; 170-15K; 172-100K; 178-33K[*]; 184-100K; 186-68K; 192-variable; 196-680K; 200-1.5K; 204-200K; 222-270K; 226-270K; 234-10.1K; 236-10.1K; 237-5K; 238-5K; 242-1M; 246-1M; 252-1M; 254-1M.

Capacitors: 104-88uf; 108-.01uf; 110-.01uf; 174-100pf; 180-100pf; 194-100pf; 202-100pf; 208-100pf; 220-3,000pf; 224-57pf; 248-433pf; 256-577pf; 258-433pf.

Driver: 106-555

Digital to Analog Converters--COMPUTER CONTINUIUM LAB 40 With a LAB 40-2 12 Bit A/d Module.

From the foregoing it can be realized that this invention provides an improved device and method for blood constituent measurement. The advantages over the prior art are: improved duty cycle, more accurate modeling by using scattering in addition to absorbance parameters, and higher accuracy in the clinical environment by accounting for red blood cell count and by filtering out ambient light, Bovie interference, and system noise. Although apparatus have been illustrated, this was for the purpose of describing, but not limiting, the invention. Various modifications, which will become apparent to one skilled in the art, are within the scope of this invention described in the attached claims.

## Claims

1. A blood constituent measuring device for non-invasive measurement of the concentration of a blood constituent by measuring the intensity of light transmitted through the body tissue of a patient at two different wavelengths, the device having;

    a light source (160, 162) for producing light of two different wavelengths,

    a driver (106, 118, 144, 148, 152, 158) having means (116, 124) for defining a first and a second clock state (Phi 1, Phi 2) and for causing the light source to produce light of one of the wavelengths during the first clock state (Phi 1) and to switch to producing light of the other wavelength during the second clock state (Phi 2), the first and second clock states (Phi 1, Phi 2) alternating periodically with one another at a given switching frequency,

    a detector (70) for detecting the light after transmission through the body tissue,

    a receiver (300, 400, 500, 600) connected to the detector (70) and to the driver to distinguish between light transmitted during the first and second clock states and to produce signals representative thereof, and

    a processor for receiving the output from the receiver (300, 400, 500, 600) and for producing an output which is indicative of the concentration of the blood constituent,

    characterized in that the driver further has means to superimpose on the light a given carrier frequency not substantially present in artificial light, the carrier frequency being superimposed on the light within each clock state, and the receiver is tuned to the carrier frequency to accept the output of the detector (70) due to said light from said source and reject interference due to artificial light.

2. A blood constituent measuring device according to claim 1, characterized in that said processor further comprises apparatus for producing at least an output which is proportional to the oxygen content of the patient's blood.

3. A blood constituent measuring device according to claim 1, characterized in that the carrier frequency of the driving signal is greater than the frequency of the fourth harmonic of the frequency of greatest amplitude in the artificial light.

4. A blood constituent measuring device according to claim 1, characterized in that the carrier frequency of the driving signal comprises a frequency which is located inbetween a pair of harmonics of the artificial light.

5. A blood constituent measuring device according to any preceding claim, characterized in that the processor comprises apparatus for generating a correction signal which is proportional to the fraction of red blood cells per unit volume of whole blood and for correcting an altered extinction of light due to scattering which is proportional to a reduced blood cell count.

6. A non-invasive method of measuring the concentration of a blood constituent by measuring the intensity of light transmitting through the body tissue of a patient at two different wavelengths comprising the steps of

    defining a first and a second clock state (Phi 1, Phi 2) and transmitting light of one of the wavelengths during

[*]Variable for gain

the first clock state (Phi 1) and transmitting along the same path light of the other wavelength during the second clock state (Phi 2), the first and second clock states (Phi 1, Phi 2) alternate periodically with one another at a given switching frequency,

detecting the light after transmission through the body tissue producing a detector output signal,

distinguishing between the detector output signal produced by light transmitted during the first and second clock states and producing signals representative thereof, and

producing from said signals an output which is indicative of the concentration of the blood constituent, characterized in superimposing on the light a given carrier frequency not substantially present in artificial light, the carrier frequency being superimposed on the light within each clock state, and there is included the step of passing the detector output signal through a receiver tuned to the given carrier frequency to accept the output of the detector (70) due to said light and reject interference due to artificial light.

7. A method for non-invasively measuring the concentration of a blood constituent according to claim 6, characterized in that the producing step produces at least an output which is proportional to the oxygen content of blood.

8. A method for non-invasively measuring the concentration of a blood constituent according to claim 6 or 7, characterized in that the producing step further comprises generating a correction signal which is proportional to the fraction of red blood cells per unit volume of whole blood and correcting for increased extinction of light due to scattering of light which is proportional to a reduced blood cell count.

**Patentansprüche**

1. Vorrichtung zur Messung von Blutbestandteilen zur nichtinvasiven Messung der Konzentration eines Blutbestandteils, indem die durch das Körpergewebe eines Patienten in zwei unterschiedlichen Wellenlängen hindurchgelassene Lichtstärke gemessen wird, wobei die Vorrichtung folgendes umfaßt:
eine Lichtquelle (160, 162) zur Erzeugung von Licht mit zwei verschiedenen Wellenlängen,
einen Treiber (106, 118, 144, 148, 152, 158), der einen ersten und einen zweiten Taktzustand (Phi 1, Phi 2) festlegt und die Lichtquelle dazu veranlaßt, Licht einer der Wellenlängen in dem ersten Taktzustand (Phi 1) zu erzeugen und auf die Erzeugung von Licht der anderen Wellenlänge in dem zweiten Taktzustand (Phi 2) umzuschalten, wobei der erste und der zweite Taktzustand (Phi 1, Phi 2) in regelmäßigen Abständen bei einer vorgegebenen Umschaltfrequenz miteinander abwechseln,
einen Detektor (70) zur Erfassung des Lichts nach der Übertragung durch das Körpergewebe,
einen Empfänger (300, 400, 500, 600), der mit dem Detektor (70) und mit dem Treiber verbunden ist, um zwischen dem im ersten Taktzustand und dem im zweiten Taktzustand hindurchgelassenen Licht zu unterscheiden, und um dies anzeigende Signale zu erzeugen, und
einen Prozessor, der das Ausgangssignal von dem Empfänger (300, 400, 500, 600) empfängt und ein Ausgangssignal absetzt, welches die Konzentration des Blutbestandteils angibt,
dadurch gekennzeichnet, daß der Treiber des weiteren eine Einrichtung umfaßt, die dem Licht eine bestimmte Trägerfrequenz, die in künstlichem Licht im wesentlichen nicht vorhanden ist, überlagert, wobei die Trägerfrequenz dem Licht in jedem Taktzustand überlagert wird, und daß der Empfänger auf die Trägerfrequenz abgestimmt ist, um das Ausgangssignal des Detektors (70) infolge des von der Lichtquelle kommenden Lichtes zu empfangen und die durch künstliches Licht bedingte Interferenz zu unterdrücken.

2. Vorrichtung zur Messung von Blutbestandteilen nach Anspruch 1, dadurch gekennzeichnet, daß der Prozessor des weiteren eine Vorrichtung umfaßt, die mindestens ein Ausgangssignal absetzt, welches proportional zum Sauerstoffgehalt des Blutes des Patienten ist.

3. Vorrichtung zur Messung von Blutbestandteilen nach Anspruch 1, dadurch gekennzeichnet, daß die Trägerfrequenz des Ansteuersignals größer ist als die Frequenz der vierten Oberschwingung der Frequenz mit der größten Amplitude beim künstlichen Licht.

4. Vorrichtung zur Messung von Blutbestandteilen nach Anspruch 1, dadurch gekennzeichnet, daß die Trägerfrequenz des Ansteuersignals eine Frequenz umfaßt, die zwischen einem Paar Oberschwingungen des künstlichen Lichts liegt.

5. Vorrichtung zur Messung von Blutbestandteilen nach einem der vorhergehenden Ansprüche, dadurch gekennzeichnet, daß der Prozessor eine Vorrichtung zur Erzeugung eines Korrektursignals umfaßt, das proportional ist zu dem Anteil von roten Blutkörperchen pro Volumeneinheit Vollblut, und zur Korrektur einer geänderten Lichtextinktion infolge Streuung, die proportional ist zu einem verminderten Anteil an roten Blutkörperchen.

6. Nichtinvasives Verfahren zur Messung der Konzentration eines Blutbestandteils durch Messen der durch das Körpergewebe eines Patienten bei zwei verschiedenen Wellenlängen hindurchgelassenen Lichtstärke, umfassend die folgenden Schritte:

Festlegen eines ersten und eines zweiten Taktzustandes (Phi 1, Phi 2) und Übertragen von Licht einer der Wellenlängen in dem ersten Taktzustand (Phi 1) und Übertragen von Licht der anderen Wellenlänge auf demselben Weg in dem zweiten Taktzustand (Phi 2), wobei der erste und der zweite Taktzustand (Phi 1, Phi 2) mit einer bestimmten Umschaltfrequenz in regelmäßigen Abständen miteinander abwechseln,

Erfassen des Lichts nach der Übertragung durch das Körpergewebe, so daß ein Detektorausgangssignal erzeugt wird,

Unterscheiden zwischen dem Detektorausgangssignal, das durch Licht erzeugt wurde, das in dem ersten und in dem zweiten Taktzustand übertragen wurde, und Absetzen von Signalen, die dies anzeigen, und

Absetzen eines Ausgangssignals auf der Basis dieser Signale, welches die Konzentration des Blutbestandteils angibt,

dadurch gekennzeichnet, daß dem Licht eine bestimmte Trägerfrequenz überlagert wird, die in künstlichem Licht im wesentlichen nicht vorhanden ist, wobei die Trägerfrequenz dem Licht in jedem Taktzustand überlagert wird, und daß der Schritt vorgesehen ist, in dem das Detektorausgangssignal durch einen Empfänger geleitet wird, der auf die vorgegebene Trägerfrequenz abgestimmt ist, um das Ausgangssignal des Detektors (70) infolge des Lichts anzunehmen und die durch künstliches Licht bedingte Interferenz zu unterdrükken.

7. Verfahren zur nichtinvasiven Messung der Konzentration eines Blutbestandteils nach Anspruch 6, dadurch gekennzeichnet, daß bei der Signalerzeugung mindestens ein Ausgangssignal erzeugt wird, das proportional ist zum Sauerstoffgehalt von Blut.

8. Verfahren zur nichtinvasiven Messung der Konzentration eines Blutbestandteils nach Anspruch 6 oder 7, dadurch gekennzeichnet, daß bei der Signalerzeugung des weiteren ein Korrektursignal erzeugt wird, das proportional ist zu dem Anteil roter Blutkörperchen pro Volumeneinheit Vollblut, und eine Korrektur der erhöhten Lichtextinktion infolge Lichtstreuung durchgeführt wird, die proportional ist zu einem verminderten Gehalt an roten Blutkörperchen.

## Revendications

1. Appareil de mesure d'éléments constituants du sang destiné à la mesure non traumatisante de la concentration d'un élément constituant du sang par mesure de l'intensité de la lumière transmise par un tissu du corps d'un patient à deux longueurs d'onde différentes, l'appareil comprenant :

une source lumineuse (160, 162) destinée à produire de la lumière à deux longueurs d'onde différentes,

un organe de pilotage (106, 118, 144, 148, 152, 158) ayant un dispositif (116, 124) destiné à définir un premier et un second état d'horloge ($\phi$1, $\phi$2) et à provoquer la création par la source lumineuse de lumière à l'une des longueurs d'onde pendant le premier état d'horloge ($\phi$1) et à commuter à la production de lumière à l'autre longueur d'onde pendant le second état d'horloge ($\phi$2), le premier et le second état d'horloge ($\phi$1, $\phi$2) alternant périodiquement à une fréquence déterminée de commutation,

un détecteur (70) destiné à détecter la lumière après transmission par le tissu du corps,

un récepteur (300, 400, 500, 600) connecté au détecteur (70) et à l'organe de pilotage et destiné à distinguer la lumière transmise au cours du premier et du second état d'horloge et à créer des signaux qui en sont représentatifs, et

un processeur destiné à recevoir le signal de sortie du récepteur (300, 400, 500, 600) et à produire un signal de sortie qui est représentatif de la concentration de l'élément constituant du sang,

caractérisé en ce que l'organe de pilotage comporte en outre un dispositif destiné à superposer à la lumière une fréquence porteuse déterminée qui n'est pratiquement pas présente dans la lumière artificielle, la fréquence porteuse étant superposée à la lumière à chaque état d'horloge, et le récepteur est accordé sur la fréquence porteuse afin qu'il accepte le signal de sortie du détecteur (70) dû à la lumière provenant de la source et rejette les interférences dues à la lumière artificielle.

2. Appareil de mesure d'éléments constituants du sang selon la revendication 1, caractérisé en ce que le processeur comporte en outre un appareil destiné à produire au moins un signal de sortie qui est proportionnel à la teneur en oxygène du sang du patient.

3. Appareil de mesure d'éléments constituants du sang selon la revendication 1, caractérisé en ce que la fréquence porteuse du signal de pilotage est supérieure à la fréquence du quatrième harmonique de la fréquence ayant la plus grande amplitude dans la lumière artificielle.

**4.** Appareil de mesure d'éléments constituants du sang selon la revendication 1, caractérisé en ce que la fréquence porteuse du signal de pilotage comprend une fréquence qui est placée entre deux harmoniques de la lumière artificielle.

**5.** Appareil de mesure d'éléments constituants du sang selon l'une quelconque des revendications précédentes, caractérisé en ce que le processeur comporte un appareil destiné à créer un signal de correction qui est proportionnel à la fraction des globules rouges par unité de volume de sang entier et à corriger l'extinction modifiée de lumière due à la diffusion qui est proportionnelle à un nombre réduit de globules rouges.

**6.** Procédé de mesure non traumatisante de la concentration d'un élément constituant du sang par mesure de l'intensité de la lumière transmise par un tissu du corps d'un patient à deux longueurs d'onde différentes, le procédé comprenant les étapes suivantes :

la définition d'un premier et d'un second état d'horloge ($\phi$1, $\phi$2) et la transmission de lumière de l'une des longueurs d'onde pendant le premier état d'horloge ($\phi$1) et la transmission le long du même trajet de lumière à l'autre longueur d'onde pendant le second état d'horloge ($\phi$2), le premier et le second état d'horloge ($\phi$1, $\phi$2) alternant périodiquement l'un avec l'autre à une fréquence donnée de commutation,

la détection de la lumière après transmission par un tissu du corps avec production d'un signal de sortie de détecteur,

la distinction entre le signal de sortie de détecteur produit par la lumière transmise pendant le premier et le second état d'horloge et la production de signaux qui en sont représentatifs, et

la production, à partir de ces signaux, d'un signal de sortie qui est représentatif de la concentration de l'élément constituant dans le sang,

caractérisé par la superposition à la lumière d'une fréquence porteuse donnée qui n'est pratiquement pas présente dans la lumière artificielle, la fréquence porteuse étant superposée à la lumière à chaque état d'horloge, et le procédé comporte une étape de transmission du signal de sortie du détecteur dans un récepteur accordé sur la fréquence porteuse donnée afin qu'il accepte le signal de sortie du détecteur (70) dû à ladite lumière et rejette les perturbations dues à la lumière artificielle.

**7.** Procédé de mesure non traumatisante de la concentration d'un élément constituant du sang selon la revendication 6, caractérisé en ce que l'étape de production assure la production d'au moins un signal de sortie qui est proportionnel à la concentration de l'oxygène dans le sang.

**8.** Procédé de mesure non traumatisante de la concentration d'un élément constituant du sang selon la revendication 6 ou 7, caractérisé en ce que l'étape de production comporte en outre la création d'un signal de correction qui est proportionnel à la fraction des globules rouges par unité de volume du sang entier, et la correction de la plus grande extinction de la lumière due à la diffusion de la lumière qui est proportionnelle à un nombre réduit de globules rouges.

GENERATE DRIVING SIGNAL AT FREQUENCY Fc WITH CONSTANT AMPLITUDE. — 2

ALTERNATELY ACTIVATE IR OR RED LED WITH DRIVING SIGNAL — 4

ADJUST LED DRIVE INTENSITY OR AMPLIFIER GAINS SO THAT THE DETECTED IR AND RED LEVELS ARE IN RANGE — 6

DETECT RECEIVED LIGHT — 7

MINIMIZE NOISE, AMBIENT LIGHT, AND BOVIE INTERFERENCE — 8

"DEMODULATE" DETECTED LIGHT FROM THE DRIVING SIGNAL — 10

DEMULTIPLEX IR AND RED DATA CHANNELS — 12

DIGITIZE IR AND RED DATA CHANNELS — 14

DETERMINE PRESENCE OF A PULSE. USE MOTION AND ARTIFACT REJECTION TECHNIQUES — 16

DETERMINE S(s) AND S(d) FOR IR AND RED CHANNELS — 18

CACULATE AND AVERAGE SATURATION — 20

DISPLAY SATURATION AND HEART RATE — 22

# FIG. 1

FIG. 2

EP 0 271 340 B1

FIG. 3

ADJUST AMPLIFIER GAIN 66 → D/A 68 → BANDPASS FILTER AT Fc 72

LINEAR PHOTODETECTOR 70 → BANDPASS FILTER AT Fc 72

FULL WAVE RECTIFIER 74

ENVELOPE DETECTOR 76

DIFFERENTIAL AMPLIFIER 78

LOW PASS FILTER 80

POSSIBLE GAIN ADJUST 86 → D/A 84 → A/D CONVERTER 82

FIG. 5

18

FIG. 4

ROOM LIGHT SPECTRUM

FIG. 7

FIG. 6

EP 0 271 340 B1